(19) Europäisches Patentamt
European Patent Office
Office européen des brevets

(11) **EP 4 414 687 A1**

(12) **EUROPEAN PATENT APPLICATION**
published in accordance with Art. 153(4) EPC

(43) Date of publication:
**14.08.2024 Bulletin 2024/33**

(21) Application number: **22889776.5**

(22) Date of filing: **19.10.2022**

(51) International Patent Classification (IPC):
**G01N 21/27** *(2006.01)*     **G01J 3/10** *(2006.01)*
**G01N 21/85** *(2006.01)*

(52) Cooperative Patent Classification (CPC):
**G01J 3/10; G01N 21/27; G01N 21/85**

(86) International application number:
**PCT/JP2022/038859**

(87) International publication number:
**WO 2023/079955 (11.05.2023 Gazette 2023/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **05.11.2021 JP 2021181094**

(71) Applicant: **Ushio Denki Kabushiki Kaisha
Tokyo 100-8150 (JP)**

(72) Inventor: **YAMADA, Go
Tokyo 100-8150 (JP)**

(74) Representative: **Maiwald GmbH
Engineering
Elisenhof
Elisenstrasse 3
80335 München (DE)**

(54) **OPTICAL MEASURING METHOD, AND OPTICAL MEASURING DEVICE**

(57)     Provided are a method and a device capable of accurately measuring an object to be measured in motion without omission, with no need for synchronizing positioning of the object to be measured and the timing of light illumination.

In an optical measurement apparatus 100, an illumination device 200 illuminates an illumination area through which an object to be measured OBJ passes, with pulsed light $S_{IN}$ whose wavelength changes with time, at a predetermined repetition frequency. A photodetector 302 receives light $S_{OBJ}$ from the object to be measured OBJ illuminated with the pulsed light $S_{IN}$. A detection signal generated by the photodetector 302 is used for calculating an optical spectrum of the object to be measured OBJ. A relational expression of

$$(f \cdot (D - d)/v) \geq 2.0 \ldots (1)$$

holds, with the repetition frequency of the pulsed light $S_{IN}$ given by $f$ [Hz], an effective size of the object to be measured OBJ in a moving direction given by $D$ [m], a moving speed of the object to be measured OBJ given by $v$ [m/s], and a size of an illumination spot of the pulsed light on a surface of the object to be measured OBJ in the moving direction given by $d$ [m].

FIG. 1

## Description

[TECHNICAL FIELD]

The present disclosure relates to an optical measurement apparatus.

[BACKGROUND ART]

Spectroscopic analysis has been widely used for component analysis and inspection of target objects. In the spectroscopic analysis, a target object is illuminated with measurement light, and a spectrum of object light affected by the target object is measured. Optical characteristic such as reflection characteristic (wavelength dependence) or transmission characteristic is obtainable, according to relationship between spectrum of the object light and spectrum of the measurement light.

Patent Literatures 1 and 2 disclose product inspection apparatuses. These product inspection apparatuses have a pulsed light source structured to illuminate a target object with pulsed light, a photodetector arranged at a position where light from the target object is incident, and a calculator structured to estimate spectral characteristic of the target object according to an output from the photodetector.

[CITATION LIST]

[Patent Literature]

[Patent Literature 1] JP 2020-159971 A
[Patent Literature 2] JP 2020-159973 A

[SUMMARY OF INVENTION]

[TECHNICAL PROBLEM]

The product inspection apparatus cannot provide accurate spectrometry, unless a timing at which the target object passes through a measurement point synchronizes with a timing of light illumination by the pulsed light source. This requires a mechanism for synchronizing the positioning with the illumination timing, and thus has complicated the device.

The present disclosure has been made considering the aforementioned problem, and an exemplary object of which is to provide a method and an apparatus capable of accurately measuring an object to be measured in motion without omission, without synchronizing positioning of the object to be measured with the timing of light illumination.

[SOLUTION TO PROBLEM]

An optical measurement method according to an aspect of the present disclosure includes: a first step of illuminating an illumination area through which an object to be measured passes with pulsed light whose wavelength changes with time, at a predetermined repetition frequency; a second step of detecting, with a photodetector, light from the object to be measured illuminated with the pulsed light in the first step; and

a third step of obtaining an optical spectrum of the object to be measured with reference to a detection signal generated by the photodetector in the second step. A relational expression of

$$(f \cdot (D - d)/v) \geq 2.0 \ ... \ (1)$$

holds, with the repetition frequency of a pulsed light given by f [Hz], an effective size of the object to be measured OBJ in a moving direction given by D [m], a moving speed of the object to be measured OBJ given by v [m/s], and a size of an illumination spot of the pulsed light on a surface of the object to be measured OBJ in the moving direction given by d [m].

Another aspect of the present disclosure relates to an optical measurement apparatus. The optical measurement apparatus includes: an illumination device structured to illuminate an illumination area through which an object to be measured passes with pulsed light whose wavelength changes with time, at a predetermined repetition frequency; and a photodetector structured to detect light from the object to be measured illuminated with the pulsed light; the optical measurement apparatus being structured to use a detection signal generated by the photodetector, for calculating an optical spectrum of the object to be measured, and to satisfy a relational expression

$$(f \cdot (D - d)/v) \geq 2.0 \ ... \ (1)$$

with a repetition frequency of the pulsed light given by f [Hz], an effective size of the object to be measured in a moving direction given by D [m], a moving speed of the object to be measured given by v [m/s], and a size of an illumination spot of the pulsed light on a surface of the object to be measured in the moving direction given by d [m].

Note also free combinations of these constituents, and also any of the constituents and expressions exchanged among the method, apparatus and system, are valid as the embodiments of the present invention.

[ADVANTAGEOUS EFFECTS OF INVENTION]

A certain embodiment of the present disclosure can provide a method and a device capable of accurately measuring an object to be measured in motion without omission, with no need for synchronizing positioning of the object to be measured and the timing of light illumination.

[BRIEF DESCRIPTION OF DRAWINGS]

[Fig. 1] Fig. 1 is a block diagram illustrating an optical measurement apparatus according to an embodiment.

[Fig. 2] Fig. 2 is a diagram explaining measurement light $S_{IN}$.

[Fig. 3] Fig. 3 is a drawing explaining spectroscopy with use of the optical measurement apparatus illustrated in Fig. 1.

[Fig. 4] Figs. 4(a) and (b) are drawings explaining light illumination in the optical measurement apparatus.

[Fig. 5] Fig. 5 is a drawing explaining illumination conditions of a pulsed light.

[Fig. 6] Fig. 6 is a drawing illustrating an exemplary pulsed light illumination onto an object to be measured.

[Fig. 7] Figs. 7(a) and (b) are charts summarizing experimental results under Condition 1 and Condition 2.

[Fig. 8] Fig. 8 is a diagram illustrating an inspection apparatus as a mode of the optical measurement apparatus.

[Fig. 9] Fig. 9 is a diagram illustrating an optical measurement apparatus according to Modified Example 1.

[Fig. 10] Fig. 10 is a diagram illustrating an optical measurement apparatus according to Modified Example 3.

[Fig. 11] Fig. 11 is a diagram illustrating an optical measurement apparatus according to Modified Example 4.

[DESCRIPTION OF EMBODIMENTS]

(Outline of Embodiments)

Some exemplary embodiments of the present disclosure will be outlined. This outline is intended for briefing some concepts of one or more embodiments, for the purpose of basic understanding of the embodiments, as an introduction before detailed description that follows, without limiting the scope of the invention or disclosure. This outline is not an extensive overview of all possible embodiments, and is therefore intended neither to specify key elements of all embodiments, nor to delineate the scope of some or all of the embodiments. For convenience, the term "one embodiment" may be used to designate one embodiment (Example or Modified Example), or a plurality of embodiments (Examples or Modified Examples) disclosed in this specification.

An optical measurement method according to one embodiment includes the following processes.

First step: Illuminating an illumination area through which an object to be measured passes with pulsed light whose wavelength changes with time, at a predetermined repetition frequency.

Second step: Detecting, with a photodetector, light from the object to be measured illuminated with the pulsed light in the first step.

Third step: Obtaining an optical spectrum of the object to be measured with reference to a detection signal generated by the photodetector in the second step.

A relational expression (1) below

$$(f \cdot (D - d)/v) \geq 2.0 \ ... \ (1)$$

holds, with the repetition frequency of the pulsed light given by f [Hz], an effective size of the object to be measured in a moving direction given by D [m], a moving speed of the object to be measured given by v [m/s], and a size of an illumination spot of the pulsed light on a surface of the object to be measured in the moving direction given by d [m].

An optical measurement apparatus according to one embodiment has an illumination device structured to illuminate an illumination area through which an object to be measured passes with pulsed light whose wavelength changes with time, at a predetermined repetition frequency, and a photodetector structured to receive light from the object to be measured illuminated with the pulsed light. A detection signal generated by the photodetector is used for calculating an optical spectrum of the object to be measured. A relational expression of

$$(f \cdot (D - d)/v) \geq 2.0 \ ... \ (1)$$

holds, with the repetition frequency of the pulsed light given by f [Hz], an effective size of the object to be measured in a moving direction given by D [m], a moving speed of the object to be measured given by v [m/s], and a size of an illumination spot of the pulsed light on a surface of the object to be measured in the moving direction given by d [m]. If the object to be measured is in accelerated motion, it suffices to employ average speed for the moving speed v [v/m].

In a case where the pulsed light is illuminated asynchronously with motion of the object to be measured in the optical measurement apparatus/method according to one embodiment, satisfaction of the relation (1) ensures that at least one shot of pulsed light is incident on the object to be measured in motion, over the duration (pulse width) of the pulse.

This enables accurate measurement of the object to be measured without omission, with no need for synchronizing positioning of the object to be measured and the timing of illumination.

Now, the "effective size" refers to the size of a part of the object to be measured that substantially takes part in the spectrometry. In an exemplary case where a part or whole of the light is shielded on the incident side of the pulsed light onto the object to be measured, or on the outgoing side of the light from the object to be measured, the effective size is determined by a region excluding the shielded part, since the shielded part does not take part in the measurement.

In one embodiment,

$$(f \cdot (D - d)/v) \geq 10.0 \ ... \ (2)$$

may hold. This ensures that at least nine shots of pulsed light are incident on the object to be measured. In this case, S/N may be improved by integrating at least nine times of output from the photodetector.

In one embodiment,

$$(f \cdot (D - d)/v) \geq 500.0 \ ... \ (3)$$

may hold. This ensures that at least 499 shots of the pulsed light are incident on the object to be measured in motion. In this case, S/N may be improved by integrating at least 499 times of output from the photodetector. This is effective in a case where the transmittance is low when measuring transmitted light through the object to be measured.

In one embodiment,

$$(f \cdot (D - d)/v) \geq 5000.0 \ ... \ (4)$$

may hold. This ensures that at least 4999 shots of the pulsed light are incident on the object to be measured in motion. In this case, S/N may be improved by integrating at least 4999 times of output from the photodetector. This is effective in a case where the transmittance is very low (10% or lower, for example) when measuring transmitted light through the object to be measured.

In one embodiment, v > 0.5 m/s may hold.

The moving speed of v > 0.5 m/s comes under the category of high speed, in usual application of spectrometry. When controlling the timing of illumination in synchronization with positioning of the object to be measured, the faster the moving speed, the more difficult the control. In contrast, this embodiment, with no need for timing adjustment, is particularly useful for applications with fast moving speed.

In one embodiment, the object to be measured may be moved by conveyance with a conveyor. This case is advantageous in terms of easiness of control of the moving speed v.

In one embodiment, the object to be measured may be moved by falling. Although the measurement of the object to be measured, while supporting it with a supporting member or the like, would undesirably shield a part of the light due to the supporting member, the present system can measure the object to be measured in a free space, thus solving the problem of shielding of light. The system can also omit any means for conveying the object to be measured, or can simplify the structure.

In one embodiment, the object to be measured may be moved by sliding on a slope. Thus the system can omit any means for conveying the object to be measured, or can simplify the structure.

In one embodiment, the illumination device may have a broadband pulsed light source, and a pulse stretcher structured to stretch a pulse width of the pulsed light emitted from the pulsed light source, so as to establish 1:1 correspondence between a wavelength and an elapsed time per pulse.

In one embodiment, the pulse stretcher may have: an array waveguide grating structured to spatially divide the pulsed light emitted from the pulsed light source corresponding to a wavelength; and a plurality of fibers in a number corresponded to the number of division of wavelength by the arrayed waveguide grating.

(Embodiments)

The present disclosure will be explained below on the basis of preferred embodiments, referring to the attached drawings. All constituents, members and processes illustrated in the individual drawings will be given same reference numerals, so as to properly avoid redundant explanations. The embodiments are merely illustrative, and are not restrictive about the disclosure. All features and combinations thereof described in the embodiments are not always essential to the disclosure.

Dimensions (thickness, length, width, etc.) of the individual members illustrated in the drawings may be appropriately enlarged or shrunk for easy understanding. Furthermore, the dimensions of the plurality of members do not necessarily indicate the dimensional relationship among them, so that a certain member A, if depicted thicker than another member B in a drawing, may even be thinner than the member B.

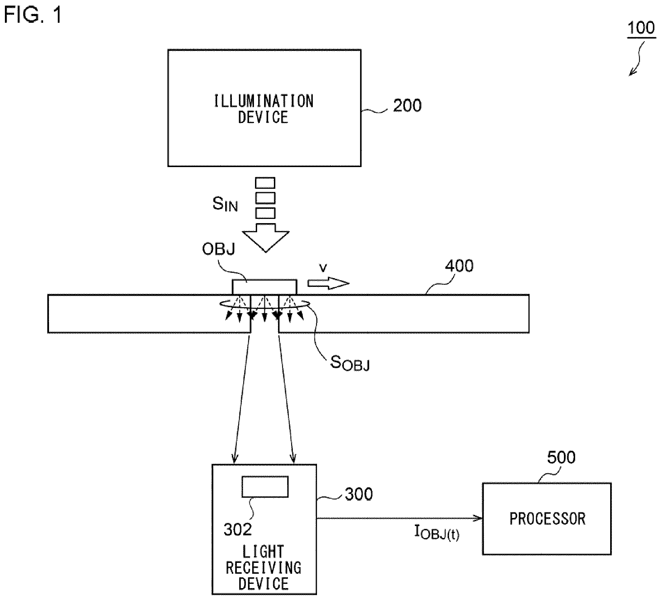

Fig. 1 is a block diagram illustrating an optical measurement apparatus 100 according to an embodiment. The optical measurement apparatus 100 is a spectrometer structured to measure a transmission spectrum of an object OBJ, and mainly includes an illumination device 200, a light receiving device 300, a conveyor 400, and a processor 500. Although some drawings will occasionally depict the illumination device 200, the light receiving device 300 or the like as a block for simplicity, this does not mean that any components constituting each device are enclosed in a single enclosure. Also note that the top, bottom, left and right in the drawings do not restrict arrangement of the individual components in the actual optical measurement apparatus 100, or the travel direction of light.

The illumination device 200 illuminates an illumination area through which an object to be measured OBJ passes, with pulsed light (referred to as measurement light, hereinafter) $S_{IN}$ whose wavelength changes with time, at a predetermined repetition frequency. The measurement light $S_{IN}$ has a 1:1 correspondence between time and wavelength. This is stated as "measurement light $S_{IN}$ has uniqueness of wavelength". The illumination device 200 may only be configured with use of any of known techniques, for which those described in Patent Literatures 1 and 2 are applicable.

The conveyor 400 supports and conveys the object to be measured OBJ. Now the verb "support" encompasses not only physically fixing the object to be measured OBJ, but also holding it within a certain range. The object to be measured OBJ moves across the illumination area, with the aid of the conveyor 400. The illumination device 200 in this embodiment operates asynchronously with the conveyance of the object to be measured OBJ with the conveyor 400, in other words, runs freely.

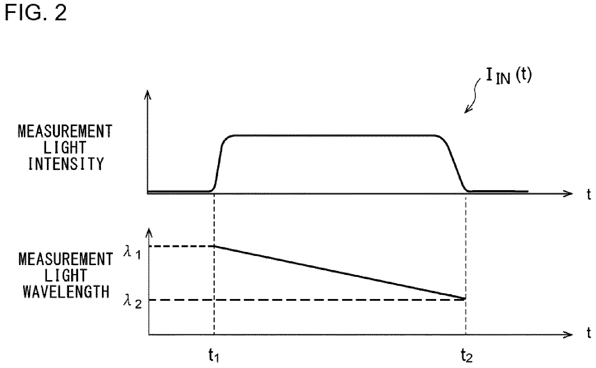

Fig. 2 is a drawing explaining the measurement light $S_{IN}$. The upper tier of Fig. 2 illustrates intensity (time waveform) $I_{IN}(t)$ of the measurement light $S_{IN}$, and the lower tier illustrates a temporal change in wavelength $\lambda$ of the measurement light $S_{IN}$.

The measurement light $S_{IN}$ in this example is given by one pulse, having dominant wavelength $\lambda_1$ at the leading edge, and dominant wavelength $\lambda_2$ at the trailing edge. The wavelength changes with time within one pulse between $\lambda_1$ and $\lambda_2$. In this example, the measurement light $S_{IN}$ is given by a positively chirped pulse ($\lambda_1 > \lambda_2$) whose frequency increases with time, in other words, whose wavelength shortens with time. Note that the measurement light $S_{IN}$ may alternatively be a negatively chirped pulse whose wavelength becomes longer with time ($\lambda_1 < \lambda_2$).

Referring now back to Fig. 1. The measurement light $S_{IN}$ is illuminated on a first face (top face) of the object to be measured OBJ, transmitted through the object to be measured OBJ, and emitted as transmitted light (also referred to as object light, hereinafter) $S_{OBJ}$ from a second face (bottom face). With a spectrum of the measurement light $S_{IN}$ given as $I_{IN}(\lambda)$, and with a wavelength dependence of the transmittance of the object light $S_{OBJ}$ given as T(A), a spectrum $I_{OBJ}(\lambda)$ of the object light $S_{OBJ}$ is given by the equation below.

$$I_{OBJ}(\lambda) = T(\lambda) \times I_{IN}(\lambda)$$

Although the object light $S_{OBJ}$ may include regular transmitted light and diffuse transmitted light, this embodiment is particularly suitable for spectrometry of an object OBJ in which the diffuse transmitted light is dominant. The regular transmitted light is emitted in the same direction as the optical axis of the measurement light $S_{IN}$, meanwhile the object light $S_{OBJ}$, which is the diffuse transmitted light, is widely emitted not only in the direction of the optical axis of the measurement light $S_{IN}$ but also in any direction different therefrom. For example, with the direction of the optical axis given by 0°, the diffuse transmitted light is emitted with an intensity distribution that follows the cosine characteristic.

The light receiving device 300 is provided on the opposite side of the illumination device 200 while placing the conveyor 400 in between, and detects the diffuse transmitted light emitted from the second face of the object to be measured OBJ. The light receiving device 300 contains a photodetector 302 structured to detect the diffuse transmitted light of the object to be measured OBJ as the object light $S_{OBJ}$. Besides the photodetector 302, the light receiving device 300 may also contain a condensing optical system or the like, which is however unillustrated in Fig. 1.

The photodetector 302 is a photoelectric conversion element that converts optical signal into electric signal, and is exemplified by photodiode, avalanche photodiode, phototransistor, photomultiplier tube making use of a photoelectric effect, and photoconductive element making use of change in electric resistance upon light illumination.

Output of the photodetector 302 is converted by an A/D converter into digital detection signal, and then supplied to a processor 500. The detection signal represents a time waveform $I_{OBJ}(t)$ of the object light $S_{OBJ}$.

The processor 500 generates a spectrum $I_{OBJ}(\lambda)$ of the object light $S_{OBJ}$, from the output signal of the light receiving device 300. The processor 500 then calculates transmittance T($\lambda$) of the object OBJ, with use of the spectrum $I_{IN}(\lambda)$ of the measurement light $S_{IN}$ and the spectrum $I_{OBJ}(\lambda)$ of the object light $S_{OBJ}$.

$$T(\lambda) = I_{OBJ}(\lambda)/I_{IN}(\lambda)$$

The spectrum $I_{IN}(\lambda)$ of the measurement light $S_{IN}$ is obtainable, alternatively by splitting a part of the measurement light $S_{IN}$ to be branched to another route, typically with use of a beam splitter on the side of the object to be measured OBJ closer to the illumination device 200, and by measuring a time waveform $I_{IN}(t)$ of the branched measurement light $S_{IN}$ with use of another light receiving device (not illustrated in Fig. 1) other than the light receiving device 300. Alternatively in a case with high stability of the measurement light $S_{IN}$, a preliminarily measured spectrum $I_{IN}(\lambda)$ may be held and used.

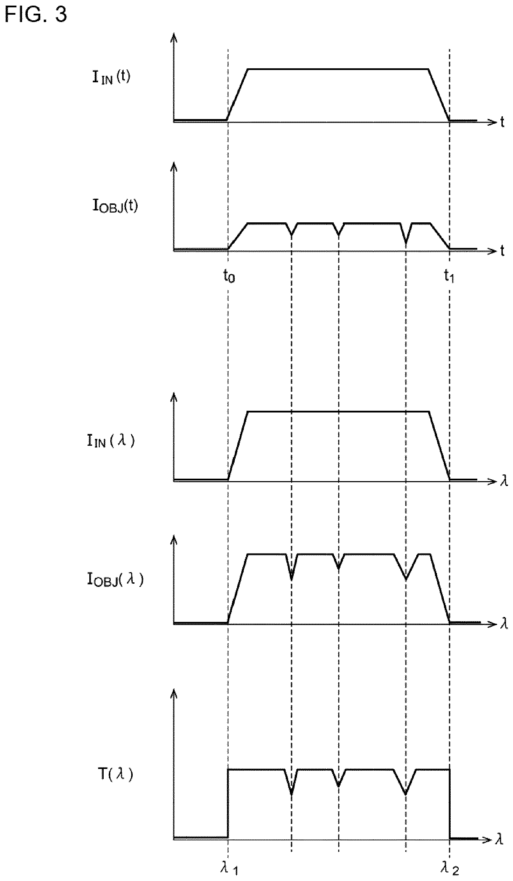

Fig. 3 is a drawing explaining spectroscopy with use of the optical measurement apparatus 100 illustrated in Fig. 1. Since the measurement light $S_{IN}$ has the 1:1 correspondence between time t and wavelength A as described previously, so that the waveform $I_{IN}$ (t) on the time-domain basis can be converted into the spectrum $I_{IN}(\lambda)$ on the frequency-domain basis.

Also the time waveform $I_{OBJ}(t)$ of the object light $S_{OBJ}$ generated from the measurement light $S_{IN}$ will have the 1:1 correspondence between time t and wavelength A. The processor 500 can therefore convert the waveform $I_{OBJ}(t)$ of the object light $S_{OBJ}$ given by the output of the light receiving device 300, into the spectrum $I_{OBJ}(\lambda)$ of the object light $S_{OBJ}$.

The processor 500 can calculate transmittance $T(\lambda)$ of the object to be measured OBJ from $I_{OBJ}(\lambda)/I_{IN}(\lambda)$, which is a ratio of two spectra $I_{OBJ}(\lambda)$ and $I_{IN}(\lambda)$.

Given that the relation between time t and wavelength A in the measurement light $S_{IN}$ is expressed by a function A = f(t). Most simply, the wavelength A varies linearly with time t according to a linear function. Lowering of the time waveform $I_{OBJ}(t)$ of the object light $S_{OBJ}$ at a certain time $t_x$ means that the transmission spectrum $T(\lambda)$ has an absorption spectrum at a wavelength $\lambda_x = f(t_x)$.

Note that the processing in the processor 500 is not limited thereto. The transmission spectrum $T(\lambda)$ may be calculated alternatively by calculating $T(t) = I_{OBJ}(t)/I_{IN}(t)$, which is the ratio of two time waveforms $I_{OBJ}(t)$ and $I_{IN}(t)$, and then by converting a variable t of the time waveform T(t) into A.

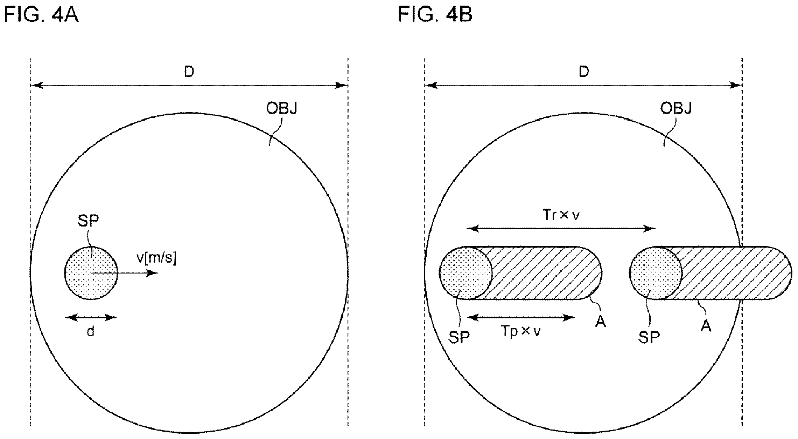

Figs. 4(a) and (b) are drawings explaining light illumination in the optical measurement apparatus 100. Fig. 4(a) illustrates the object to be measured OBJ and an instantaneous illumination spot SP of the measurement light $S_{IN}$. Although the object to be measured OBJ actually moves while keeping the illumination spot SP stationary, the description herein will be made on relative movement in which the illumination spot SP moves. While the object to be measured OBJ and the illumination spot SP herein will be depicted with circles, the shapes thereof are not particularly limited.

Letting the effective size in the moving direction (rightward in the drawing) of the object to be measured OBJ be D [m], and the moving speed of the object to be measured OBJ be v [m/s]. Referring to Fig. 1, the object light $S_{OBJ}$ emitted through the bottom face of the object to be measured OBJ is shielded by the conveyor 400. That is, the shielded area of the object to be measured OBJ does not take part in the measurement. The effective size D of the object to be measured OBJ in this case is given by the width of the opening of the conveyor 400.

If the conveyor 400 illustrated in Fig. 1 has a transparent part that supports and conveys the object to be measured OBJ, the effective size of the object to be measured OBJ will coincide with the actual size of the object to be measured OBJ.

Referring now back to Figs. 4(a) and (b). Letting the size of the illumination spot of the measurement light $S_{IN}$ in the moving direction, on the face of the object to be measured OBJ, be d [m]. Assume that a relational expression d < D holds. Fig. 4 (b) illustrates an illumination area A formed by the pulsed light. With the pulse width of the measurement light given by Tp [s], the width of the illumination area A linearly extends with respect to the pulse width Tp.

Fig. 4 (b) illustrates illumination areas A formed by two temporally adjacent pulses. The interval between the two illumination areas A becomes longer, corresponding to the repetition period Tr = 1/f [s] of the pulsed light. Now, f [Hz] represents the repetition frequency of the pulsed light. The pulse width Tp of the measurement light is smaller than the period Tr of the light illumination, between which the relation below holds.

$$Tp < Tr$$

Tp/Tr is also referred to as duty cycle.

The illumination device 200 in this embodiment operates asynchronously with the conveyance of the object to be measured OBJ with the conveyor 400, as described previously. In this case, at least one shot of pulsed light is necessarily illuminated on the object to be measured OBJ in motion without going out thereof in any situation, over the duration of the pulse (pulse width). In other words, as illustrated in Fig. 4(b), at least one illumination area A should completely fall within the object to be measured OBJ without going out thereof. Conditions required herein will be described.

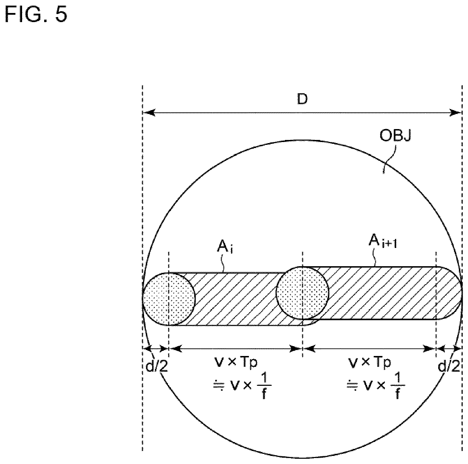

Fig. 5 is a drawing explaining illumination conditions of the pulsed light. Consider now a case where the duty cycle of the pulsed measurement light $S_{IN}$ is infinitely close to 1 (that is, Tp ≈ Tr). Fig. 5 illustrates a state in which both illumination areas $A_i$ and $A_{i+1}$ formed by two (K = 2) temporally sequential shots of pulsed light fall within the object to be measured OBJ without going out thereof. In this state, the following equation holds.

$$(f \cdot (D - d)/v) = 2.0$$

In this case, two (K = 2) shots of pulsed light are used for the spectrometry.

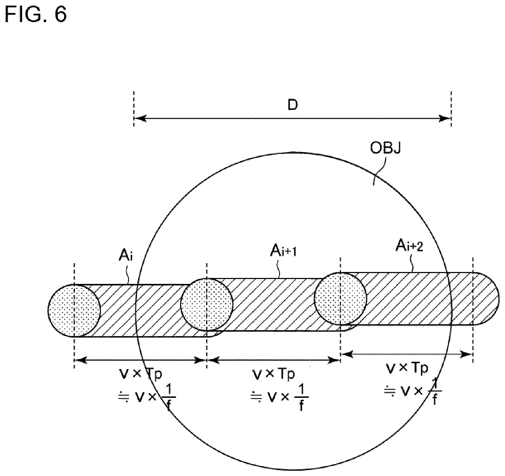

Fig. 6 is a drawing illustrating an exemplary pulsed light illumination onto the object to be measured. Since the conveyance of the object to be measured OBJ and the illumination of the pulsed light are asynchronous, so that the positional relation between the illumination area A and the object to be measured OBJ drifts in the left-right direction, as illustrated in Fig. 6. Fig. 6 illustrates illumination areas $A_i$, $A_{i+1}$, and $A_{i+2}$ formed by three (= K + 1) temporally sequential shots of pulsed light. In a case where the aforementioned equation holds, the center illumination area $A_{i+1}$, from among the three sequential illumination areas $A_i$, $A_{i+1}$ and $A_{i+2}$, completely falls within

the object to be measured OBJ, even if the timing of shot of the pulsed light were shifted ahead or behind on the time axis.

In the situation illustrated in Fig. 6, the illumination area $A_i$ which partially goes out of the object to be measured OBJ is considered to lack the first half of the time waveform $I_{OBJ}(t)$ of the object light $S_{OBJ}$ illustrated in Fig. 3, or to lack information of the object to be measured OBJ. Similarly, also the illumination area $A_{i+2}$ is considered to lack the latter half of the time waveform $I_{OBJ}(t)$ of the object light $S_{OBJ}$ illustrated in Fig. 3, or to lack information of the object to be measured OBJ.

The time waveforms $I_{OBJ}(t)$ of the object light $S_{OBJ}$ which correspond to the illumination areas $A_i$ and $A_{i+2}$ are, therefore, preferably omitted, rather than being incorporated in the spectrometry. A method of omitting the improper time waveforms $I_{OBJ}(t)$ is not limited. For example, the improper time waveforms $I_{OBJ}(t)$ may be left uncaptured by an A/D converter. Alternatively, the improper time waveforms $I_{OBJ}(t)$ may be captured by the A/D converter, and then omitted from the integration by way of signal processing. Whether a certain time waveform $I_{OBJ}(t)$ is improper or not may be judged from the waveform. Alternatively, the judgment may be made by monitoring the position of the object to be measured OBJ and the position of the illumination area A.

In this way, the situation illustrated in Fig. 6 can employ the center one of the three temporally sequential shots of pulsed light for the spectrometry.

The discussion above teaches that a condition under which one illumination area A always falls within the object to be measured OBJ in any situation, in other words, a condition under which at least one shot of pulsed light is illuminated on the object to be measured OBJ over the duration of the pulse (pulse width) Tp, is given by

$$(f \cdot (D - d)/v) \geq 2.0 \ ... \ (1).$$

With f, D, d and v specified so as to satisfy the relational expression (1), it now becomes possible to accurately measure the object to be measured OBJ in motion without omission, with no need for synchronizing positioning of the object to be measured and the timing of illumination.

The expression (1) is modified to obtain expression (1a) that describes allowable frequency range.

$$f \geq 2.0 \times v/(D - d) \ ...(1a)$$

While the discussion above has dealt with a condition under which one object to be measured OBJ is reliably illuminated with one shot of pulsed light, the expression (1a) can be generalized by incorporating an arbitrary constant K (K ≥ 2) to obtain equation (1b).

$$f = K \times v/(D - d) \ ...(1b)$$

With the repetition frequency f specified so as to satisfy the equation (1b), it is ensured that at least (K - 1) shots of pulse are illuminated on the object to be measured OBJ, over the entire ranges of the individual pulse widths. As the constant K made larger, the number of shots of the pulsed light incident on one object to be measured OBJ increases, thus improving $S_{IN}$.

For example, with K ≥ 10, then relational expression (2) is given as a conditional expression.

$$(f \cdot (D-d)/v) \geq 10.0 \ ...(2)$$

This ensures that at least nine shots of pulsed light are incident on the object to be measured. In this case, S/N may be improved by integrating at least nine times of output from the photodetector. Meanwhile with small K, the measurement is suitable for obtaining reflection spectrum.

For example, with K ≥ 500, then relational expression (3) is given as a conditional expression.

$$(f \cdot (D - d)/v) \geq 500.0 \ ...(3)$$

This ensures that at least 499 shots of the pulsed light are incident on the object to be measured in motion. In this case, S/N may be improved by integrating at least 499 times of output from the photodetector. This is effective in a case where the transmittance is low when measuring transmitted light through the object to be measured OBJ.

For example, with K ≥ 5000, then relational expression (4) is given as a conditional expression.

$$(f \cdot (D - d)/v) \geq 5000.0 \ ...(4)$$

This ensures that at least 4999 shots of the pulsed light are incident on the object to be measured in motion. In this case, S/N may be improved by integrating at least 4999 times of output from the photodetector. This is effective in a case where the transmittance is very low (10% or lower, for example) when measuring transmitted light through the object to be measured OBJ.

The structure and operation of the optical measurement apparatus 100 have been described. Next, experiments with use of the optical measurement apparatus 100 will be described.

(Experimental Conditions)

The illumination device 200 is a pulse spectroscopic light source equipped with an array waveguide grating (AWG). The pulsed light $S_{IN}$ has a wavelength of 900 to 1300 nm, and a stretching time of 0.5 μs (50 channels at 2 m intervals). The repetition frequency f of the pulsed light is variable.

The experiment was conducted with use of 100 object to be measured OBJ (n = 100), under two types of conditions 1 and 2 below.

(Condition 1)

Size D of the object to be measured OBJ in the moving direction (a part exposed as a face to be illuminated): 11 mm

Spot size d of the pulsed light on the face of the object to be measured OBJ: 1 mm
Moving speed v of the object to be measured OBJ: 1 m/s
Intervals between the object to be measured OBJ: 1.57 mm

A detection signal of the photodetector 302 was acquired with a digitizer, a gate signal (width = 11 msec, period = 12.57 msec) synchronized with the movement of the object to be measured OBJ was input to the digitizer, and data was collected during ON-time of the gate signal, while triggered with a seed laser, at a capture time of 0.5 psec.

(Condition 2)

Size D of the object to be measured OBJ in the moving direction (a part exposed as a face to be illuminated): 1.1 mm Spot size d of the pulsed light on the face of the object to be measured OBJ: 0.1 mm

Moving speed v of the object to be measured OBJ: 10 m/s
Intervals between the object to be measured OBJ: 11.47 mm

Under condition 2, a gate signal having a width of 0.11 msec and a period of 1.257 msec was used. The capture time was 0.5 psec.

(Experimental Results)

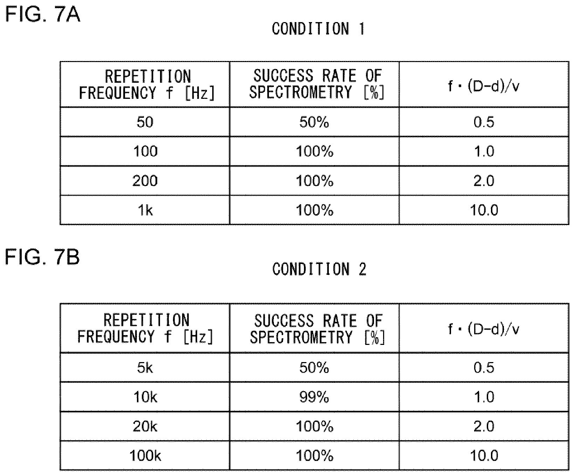

Figs. 7(a) and (b) are chart summarizing experimental results obtained under Condition 1 and Condition 2. Reference will now be made on Fig. 7(a). Fifty percent (50 pieces) of the object to be measured OBJ failed to yield accurate spectra at f = 50 Hz, whereas all of 100 pieces of the object to be measured OBJ accurately went through the spectrometry at 100 Hz, 200 Hz, and 1 kHz.

Reference will then be made on Fig. 7(b). Fifty pieces of the object to be measured OBJ failed to yield accurate spectra at f = 5 kHz, and one piece of the object to be measured OBJ failed to yield accurate spectra at f = 10 kHz. Accurate spectra were obtained at f = 20 kHz and 100 kHz.

These experimental results support the validity of the relational expression (1).

(Applications)

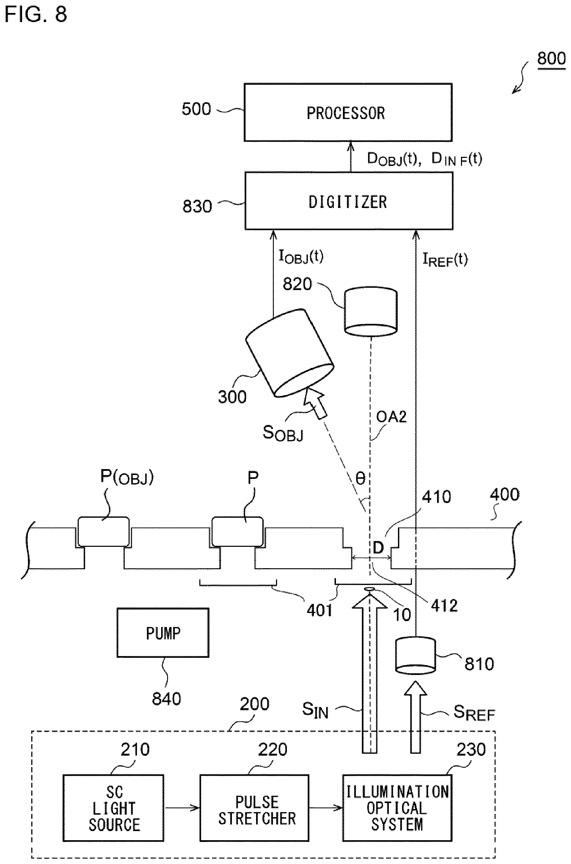

Next, applications of the optical measurement apparatus 100 according to the embodiment will be explained. The optical measurement apparatus 100 is applicable to an inspection apparatus typically for food and beverage products having been powdered and solidified. Fig. 8 is a diagram illustrating an inspection apparatus 800 as a mode of the optical measurement apparatus 100. The inspection apparatus 800 inspects a large amount of products P such as food and beverage, and determines the quality. Food and beverage have a transmittance on the order of 1/100 to 1/1000.

As has been described regarding the optical measurement apparatus 100, the inspection apparatus 800 has the illumination device 200, the light receiving device 300, the conveyor 400, and the processor 500. The inspection apparatus 800 additionally has a light receiving device 810, a beam damper 820, a digitizer 830, and a pump 840.

The illumination device 200 has a light source 210, a pulse stretcher 220, and an illumination optical system 230. The light source 210 produces a continuous spectrum of at least 10 nm bandwidth, and more specifically produces a broad coherent pulsed light in an infrared region from 900 to 1300 nm. The light source 210 may be a super continuum (SC) light source having a pulsed laser and a non-linear element. The pulsed laser usable here includes mode locked laser, microchip laser, and fiber laser. The non-linear element usable here includes non-linear fiber such as photonic crystal fiber.

The pulse stretcher 220 stretches the pulse width of the pulsed light generated by the light source 210, so as to establish the 1:1 correspondence between time and wavelength. The pulse stretcher 220 may be constituted by a single wavelength dispersion fiber.

Alternatively, the pulse stretcher 220 may be constituted by a demultiplexer that branches the pulsed light into a plurality of paths wavelength by wavelength, a plurality of fibers (a fiber bundle) that gives different delays to the plurality of paths, and a multiplexer that recombines outputs of the plurality of fibers. The demultiplexer may be constituted by a planar lightwave circuits (PLC), and more specifically by an array waveguide grating (AWG). The individual fibers that constitute the fiber bundle have different lengths.

In the conveyor 400, each support part 401 has a recess 410. A plurality of products P are placed in the recesses 410 with use of a mounter (not illustrated), on the upstream side (left side in the drawing). The conveyor 400 conveys the plurality of support parts 401 in the direction of arrangement thereof (rightwards in the drawing). Of the faces of the recess 410, the surface on which the product P is placed will be referred to as a top face, and the opposite surface will be referred to as a back face.

The illumination optical system 230 illuminates the illumination region 10 with stretched pulses used as the measurement light $S_{IN}$. The illumination region 10 is situated at a point where the product P passes, that is, where the recess 410 passes. The illumination optical system 230 may be constituted by a transmission optical system such as a lens, a reflection optical system such as a mirror, or a combination thereof. With movement of the recess 410, the products P sequentially pass across the illumination region 10.

The light source 210 repeatedly generates the pulsed light at a predetermined frequency (period). Operating frequency of the light source 210 may only be determined according to the moving speed of the recess 410, or, the conveying speed of the products P, so that a plurality of shots of measurement light $S_{IN}$ will be illuminated on the same product P while the product P resides in the illumination region 10. More specifically, the repetition frequency f is determined so as to satisfy the aforementioned relational expression (1).

Operation of the light source 210 is independent of operation of the conveyor 400, in other words, position of the products P. The measurement light $S_{IN}$ is, therefore, repeatedly illuminated on the illumination region 10, even if the product P is not placed in the recess 410.

The light receiving device 300 is provided above the recess 410. The recess 410 has a through-hole 412 formed in the bottom face. The through-hole 412 is formed to guide the measurement light $S_{IN}$ from the illumination optical system 230, to the bottom face of the product P. In this structure, a part of the product P as the object to be measured, which takes part in the spectrometry, in other words, a part exposed to be illuminated by the measurement light $S_{IN}$ (pulsed light), corresponds to the through-hole 412. Hence, the size (diameter) of the through-hole 412, rather than the size (diameter) of the product P, gives the effective size D of the product P.

A pump 840 may be provided on the back side of the recess 410. The pump 840 constitutes a suction unit, with which the back side of the recess 410 is evacuated to produce a negative pressure, making the product P adhere to the recess 410, thereby preventing the product P from falling off from the recess 410 as the product P is conveyed.

The light receiving device 300 measures a time waveform $I_{OBJ}(t)$ of the object light $S_{OBJ}$. On the optical axis OA2 of the measurement light $S_{IN}$, there is also provided a beam damper 820 to prevent stray light.

The light receiving device 810 is provided to measure the spectrum of the measurement light $S_{IN}$. The illumination optical system 230 branches a part of the measurement light $S_{IN}$ into a separate arm as the reference light $S_{REF}$, typically with use of a beam splitter. The light receiving device 810 measures a time waveform $I_{REF}(t)$ of the reference light $S_{REF}$ branched into the separate arm. The time waveform $I_{REF}(t)$ is equivalent to the time waveform $I_{IN}(t)$ of the measurement light $S_{IN}$.

The digitizer 830, having an A/D converter, samples outputs of the light receiving device 300 and the light receiving device 810, that is, time waveforms $I_{OBJ}(T)$ and $I_{REF}(t)$, respectively, at a predetermined sampling frequency, and converts them respectively into waveform data $D_{OBJ}(t)$ and $D_{IN}(t)$ of digital signals.

The digitizer 830 is omissible, when using the light receiving devices 300 and 810 designed for digital output.

The processor 500 processes the digital waveform data $D_{OBJ}(t)$ and $D_{IN}(t)$ to obtain a transmission characteristic (or absorption characteristic) $T(\lambda)$ of the product P. The processor 500 may be implemented by a general-purpose or dedicated computer having a processor, a memory, and a storage medium such as hard disk, while combined with software program. The processing by the processor 500 is as described above.

With the inspection apparatus 800, the transmission spectrum of the product P may be accurately measured, without synchronizing the timing of conveyance of the product P with the timing of light illumination by the illumination device 200.

With the inspection apparatus 800 thus structured to illuminate the product P with the measurement light $S_{IN}$ from behind the conveyor 400, the object light $S_{OBJ}$ may be prevented from being shielded by the through-hole 412, thereby enabling detection of much more object light $S_{OBJ}$ by the light receiving device 300.

Note in the inspection apparatus 800 illustrated in Fig. 8, with no product P placed in the recess 410, the measurement light $S_{IN}$ will leak through the through-hole 412 towards the light receiving device 300. Assuming a case in which the light receiving device 300 were arranged on the optical axis OA2 of the measurement light $S_{IN}$, and no product P were placed on the optical axis OA2, the high-intensity measurement light $S_{IN}$ would be incident directly on the photodetector 302. This is not desirable. Hence, the light receiving device 300 is preferably structured to avoid incidence of the measurement light $S_{IN}$ on the photodetector 302, when there is no product P placed in the recess 410.

For example, the light receiving device 300 is structured so that a component $S_{\theta}$ of the diffuse transmitted light (object light $S_{OBJ}$) of the object to be measured OBJ, which is emitted in a direction deviated from the optical axis OA2 of the measurement light $S_{IN}$ (with deviation angle θ), is incident on the photodetector 302.

It now suffices that the object light $S_{OBJ}$ in the direction of the optical axis OA2 will not be incident on the photodetector 302, while allowing incidence on an incident aperture of the light receiving device 300.

The light receiving device 300 may thus be protected even in the absence of the product P in the recess 410. The light source 210 of the illumination device 200 in this case may be allowed to freely run asynchronously with the operation of the conveyor 400, and also there is no need for shutter control in synchronization with the operation of the conveyor 400.

Next, Modified Examples will be described.

(Modified Example 1)

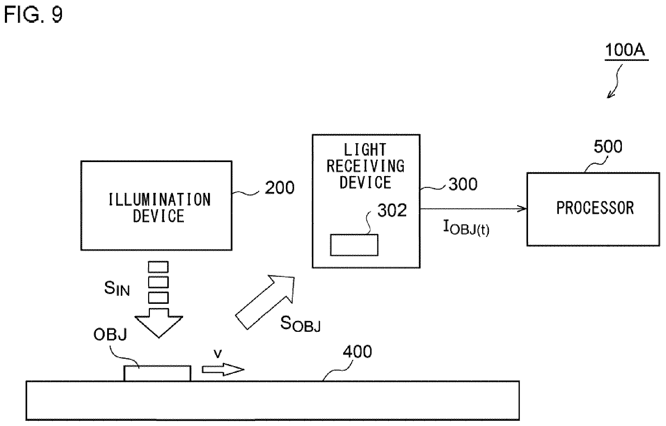

Although the transmission spectrum of the object to be measured OBJ was measured in the embodiment, reflection spectrum may alternatively be measured. Fig. 9 is a diagram illustrating an optical measurement apparatus 100A according to Modified Example 1. The light receiving device 300 is arranged to detect reflected light on the object to be measured OBJ as the object light $S_{OBJ}$. In the case illustrated in Fig. 9, neither the measurement light $S_{IN}$ nor the object light $S_{OBJ}$ is shielded, so that the effective size of the object to be measured OBJ coincides with the actual size of the object to be measured OBJ.

(Modified Example 2)

Although the product P as the object to be measured OBJ was placed in the recess 410 in Fig. 8, a mode of supporting and transfer of the product P is not limited thereto. The product P may move while being placed on a roller conveyor or on a simple belt. In this case, the intervals between the adjacent products P will become non-uniform, and this makes it difficult to predict the time the object to be measured OBJ passes through the illumination region. Even in such situation, the spectrum of the object to be measured OBJ may be accurately measured, by determining the illumination repetition frequency f so as to satisfy the expression (1).

(Modified Example 3)

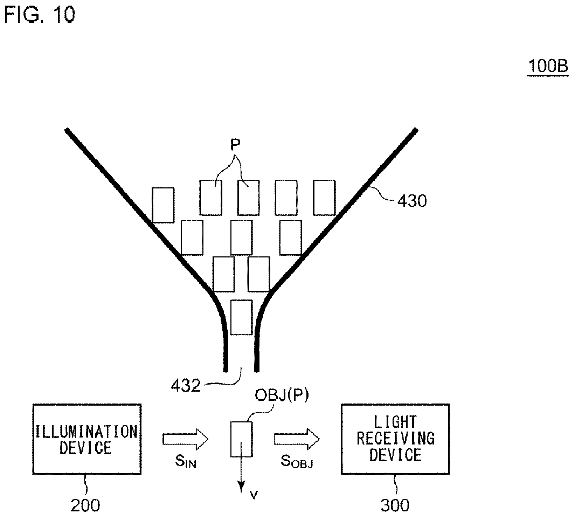

Fig. 10 is a diagram illustrating an optical measurement apparatus 100B according to Modified Example 3. The optical measurement apparatus 100B has a drop-type transfer device 430. The object to be measured OBJ falls from a drop port 432 of the transfer device 430. The object to be measured OBJ, when freely falls, is in accelerated motion.

The illumination device 200 illuminates the object to be measured OBJ, during falling in a free space, with the measurement light $S_{IN}$ at the repetition frequency f. The light receiving device 300 measures the transmitted light or reflected light from the object to be measured OBJ. In this case, the velocity v in the expression (1) may be an average speed or a maximum speed when intercepting the measurement light $S_{IN}$. In the case illustrated in Fig. 10, neither the measurement light $S_{IN}$ nor the object light $S_{OBJ}$ is shielded, so that the effective size of the object to be measured OBJ coincides with the actual size of the object to be measured OBJ.

Modified Example 3 can measure the object to be measured OBJ in the free space, and can therefore solve the problem of light shielding. Another advantage is that the means for conveying the object to be measured OBJ is omissible, or that the structure may be simplified.

(Modified Example 4)

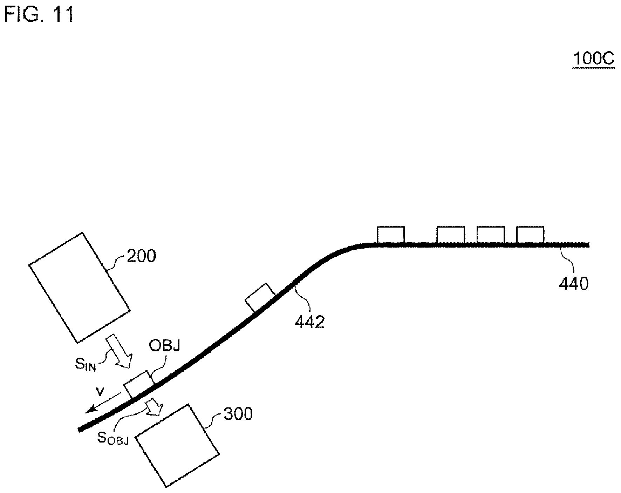

Fig. 11 is a diagram illustrating an optical measurement apparatus 100C according to Modified Example 4. The optical measurement apparatus 100C has a transfer device 440. The transfer device 440 has a slope 442. The object to be measured OBJ slides on the slope 442 so as to move across the illumination range of the illumination device 200.

The light receiving device 300 measures the transmitted light or reflected light from the object to be measured OBJ. For a case where the object to be measured OBJ is in accelerated motion, the velocity v in the expression (1) may only be an average speed or a maximum speed when intercepting the measurement light $S_{IN}$.

[REFERENCE SIGNS LIST]

OBJ object to be measured, P product, 100 optical measurement apparatus, 200 illumination device, 210 light source, 220 pulse stretcher, 230 illumination optical system, 300 light receiving device, 302 photodetector, 400 conveyor, 401 support part, 410 recess, 412 through-hole, 430 transfer device, 432 drop port, 440 transfer device, 442 slope, 500 processor, 800 inspection apparatus, 810 light receiving device, $S_{IN}$ measurement light, $S_{OBJ}$ object light, 820 beam damper, 830 digitizer, 840 pump

**Claims**

1. An optical measurement method comprising:

   a first step of illuminating an illumination area through which an object to be measured passes with pulsed light whose wavelength changes with time, at a predetermined repetition frequency;
   a second step of detecting, with a photodetector, light from the object to be measured illuminated with the pulsed light in the first step; and
   a third step of obtaining an optical spectrum of the object to be measured with reference to a detection signal generated by the photodetector in the second step,
   the method being structured to satisfy a relational expression

   $$(f \cdot (D - d)/v) \geq 2.0 \ ... \ (1)$$

   with a repetition frequency of the pulsed light given by f [Hz], an effective size of the object to be measured in a moving direction given by D [m], a moving speed of the object to be measured given by v [m/s], and a size of an illumination spot of the pulsed light on a surface of the object to be measured in the moving direction given by d [m].

2. The optical measurement method according to claim 1, being structured to satisfy a relational expression.

$$(f \cdot (D - d)/v) \geq 10.0 \dots (2)$$

3. The optical measurement method according to claim 1, being structured to satisfy a relational expression.

$$(f \cdot (D - d)/v) \geq 500.0 \dots (3)$$

4. The optical measurement method according to claim 1, being structured to satisfy a relational expression.

$$(f \cdot (D - d)/v) \geq 5000.0 \dots (4)$$

5. The optical measurement method according to any one of claims 1 to 4, wherein v > 0.5 m/s.

6. The optical measurement method according to any one of claims 1 to 4, wherein the object to be measured moves while conveyed with a conveyor.

7. The optical measurement method according to any one of claims 1 to 4, wherein the object to be measured moves by falling.

8. The optical measurement method according to any one of claims 1 to 4, wherein the object to be measured moves by sliding on a slope.

9. An optical measurement apparatus comprising:

an illumination device structured to illuminate an illumination area through which an object to be measured passes with pulsed light whose wavelength changes with time, at a predetermined repetition frequency; and
a photodetector structured to detect light from the object to be measured illuminated with the pulsed light;
the optical measurement apparatus being structured to use a detection signal generated by the photodetector, for calculating an optical spectrum of the object to be measured, and to satisfy a relational expression

$$(f \cdot (D - d)/v) \geq 2.0 \dots (1)$$

with a repetition frequency of the pulsed light given by f [Hz], an effective size of the object to be measured in a moving direction given by D [m], a moving speed of the object to be measured given by v [m/s], and a size of an illumination spot of the pulsed light on a surface of the object to be measured in the moving direction given by d [m].

10. The optical measurement apparatus according to claim 9, being structured to satisfy a relational expression.

$$(f \cdot (D - d)/v) \geq 10.0 \dots (2)$$

11. The optical measurement apparatus according to claim 9, being structured to satisfy a relational expression.

$$(f \cdot (D - d)/v) \geq 500.0 \dots (3)$$

12. The optical measurement apparatus according to claim 9, being structured to satisfy a relational expression.

$$(f \cdot (D - d)/v) \geq 5000.0 \dots (4)$$

13. The optical measurement apparatus according to any one of claims 9 to 12, wherein v > 0.5 m/s.

14. The optical measurement apparatus according to any one of claims 9 to 12, wherein the illumination device includes a broadband pulsed light source, and a pulse stretcher structured to stretch a pulse width of the pulsed light emitted from the pulsed light source, so as to establish a 1:1 correspondence between a wavelength and an elapsed time per pulse.

15. The optical measurement apparatus according to claim 14, wherein the pulse stretcher includes:

an array waveguide grating structured to spatially divide the pulsed light emitted from the pulsed light source corresponding to wavelength; and
a plurality of fibers in a number corresponded to the number of division of wavelength by the arrayed waveguide grating.

16. The optical measurement apparatus according to any one of claims 9 to 12, wherein the object to be measured moves by falling.

17. The optical measurement apparatus according to any one of claims 9 to 12, wherein the object to be measured moves by sliding on a slope.

EP 4 414 687 A1

FIG. 1

EP 4 414 687 A1

FIG. 2

FIG. 3

FIG. 4A

FIG. 4B

EP 4 414 687 A1

FIG. 5

FIG. 6

FIG. 7A

CONDITION 1

| REPETITION FREQUENCY f [Hz] | SUCCESS RATE OF SPECTROMETRY [%] | $f \cdot (D-d)/v$ |
|---|---|---|
| 50 | 50% | 0.5 |
| 100 | 100% | 1.0 |
| 200 | 100% | 2.0 |
| 1k | 100% | 10.0 |

FIG. 7B

CONDITION 2

| REPETITION FREQUENCY f [Hz] | SUCCESS RATE OF SPECTROMETRY [%] | $f \cdot (D-d)/v$ |
|---|---|---|
| 5k | 50% | 0.5 |
| 10k | 99% | 1.0 |
| 20k | 100% | 2.0 |
| 100k | 100% | 10.0 |

FIG. 8

FIG. 9

EP 4 414 687 A1

EP 4 414 687 A1

FIG. 10

100B

EP 4 414 687 A1

FIG. 11

100C

## INTERNATIONAL SEARCH REPORT

| International application No. |
|---|
| **PCT/JP2022/038859** |

### A. CLASSIFICATION OF SUBJECT MATTER

*G01N 21/27*(2006.01)i; *G01J 3/10*(2006.01)i; *G01N 21/85*(2006.01)i
FI:   G01N21/27 A; G01J3/10; G01N21/85 Z

According to International Patent Classification (IPC) or to both national classification and IPC

### B. FIELDS SEARCHED

Minimum documentation searched (classification system followed by classification symbols)

G01N 21/00-21/01; G01N 21/17-21/61; G01J 1/02-1/04; G01J 1/08; G01J 3/00-3/51; G01J 11/00; G01N 21/84-21/958; G01B 11/00-11/30; A61J 3/06; B07C 5/342

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2015-049062 A (HITACHI HIGH-TECHNOLOGIES CORPORATION) 16 March 2015 (2015-03-16)<br>paragraphs [0014]-[0020], [0032], fig. 1, 4 | 1-6, 9-13 |
| Y | | 7-8, 16-17 |
| Y | paragraphs [0024]-[0029], fig. 3 | 14-15 |
| Y | JP 2016-537646 A (TOMRA SORTING NV) 01 December 2016 (2016-12-01)<br>paragraphs [0039]-[0050], fig. 1a-2 | 7, 16 |
| Y | JP 2010-110733 A (MITSUBISHI ELECTRIC CORP) 20 May 2010 (2010-05-20)<br>paragraphs [0011]-[0013], fig. 1-2 | 8, 17 |
| Y | WO 2020/196690 A1 (USHIO ELECTRIC INC) 01 October 2020 (2020-10-01)<br>paragraphs [0076]-[0078], fig. 10 | 14-15 |
| P, X | WO 2022/064874 A1 (USHIO ELECTRIC INC) 31 March 2022 (2022-03-31)<br>paragraphs [0013]-[0027], [0046]-[0050], fig. 1-3, 7 | 1-2, 5-6, 9-10, 14-15 |

☑ Further documents are listed in the continuation of Box C.     ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **01 December 2022** | **13 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**EP 4 414 687 A1**

<table>
<tr><td colspan="2"><strong>INTERNATIONAL SEARCH REPORT</strong></td><td>International application No.<br><strong>PCT/JP2022/038859</strong></td></tr>
</table>

**C. DOCUMENTS CONSIDERED TO BE RELEVANT**

| Category* | Citation of document, with indication, where appropriate, of the relevant passages | Relevant to claim No. |
|---|---|---|
| A | WO 2014/097943 A1 (TORAY INDUSTRIES, INC.) 26 June 2014 (2014-06-26)<br>paragraph [0059] | 1-17 |
| A | JP 2007-057360 A (AGILENT TECHNOLOGIES INC) 08 March 2007 (2007-03-08)<br>paragraph [0029], fig. 1 | 1-17 |
| A | JP 07-099713 A (KOMATSU MFG CO LTD) 11 April 1995 (1995-04-11)<br>paragraphs [0045]-[0047] | 1-17 |
| E, A | JP 2022-160819 A (USHIO ELECTRIC INC) 20 October 2022 (2022-10-20)<br>paragraphs [0068]-[0083], fig. 11 | 1-17 |

Form PCT/ISA/210 (second sheet) (January 2015)

24

## INTERNATIONAL SEARCH REPORT
### Information on patent family members

International application No.

**PCT/JP2022/038859**

| Patent document cited in search report | | | Publication date (day/month/year) | Patent family member(s) | | | Publication date (day/month/year) |
|---|---|---|---|---|---|---|---|
| JP | 2015-049062 | A | 16 March 2015 | US 2015/0062581 A1 paragraphs [0025]-[0031], [0035]-[0040], [0043], fig. 1, 3-4 | | | |
| JP | 2016-537646 | A | 01 December 2016 | US 2016/0263624 A1 paragraphs [0060]-[0071], fig. 1a-2 WO 2015/063299 A1 | | | |
| JP | 2010-110733 | A | 20 May 2010 | (Family: none) | | | |
| WO | 2020/196690 | A1 | 01 October 2020 | JP 2020-159971 A paragraphs [0076]-[0078], fig. 10 US 2022/0178848 A1 paragraphs [0145]-[0149], fig. 10 EP 3951366 A1 | | | |
| WO | 2022/064874 | A1 | 31 March 2022 | JP 2022-052371 A paragraphs [0013]-[0027], [0046]-[0050], fig. 1-3. 7 | | | |
| WO | 2014/097943 | A1 | 26 June 2014 | US 2015/0293025 A1 | | | |
| JP | 2007-057360 | A | 08 March 2007 | US 2007/0097372 A1 DE 102006039670 A1 CN 1932475 A | | | |
| JP | 07-099713 | A | 11 April 1995 | (Family: none) | | | |
| JP | 2022-160819 | A | 20 October 2022 | WO 2022/215453 A1 | | | |

Form PCT/ISA/210 (patent family annex) (January 2015)

**EP 4 414 687 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020159971 A

- JP 2020159973 A